# EUROPEAN PATENT APPLICATION

(11) **EP 2 208 500 A1**
(43) Date of publication of application: **21.07.2010**
(21) Application number: 09425009.9
(22) Date of filing: 16.01.2009
(51) Int. Cl.: A61K 36/60, A61P 1/00

(54) **Compositions for the treatment of gerd (gastroesophageal reflux disease)**

(71) Applicant: Bionap S.r.L., 95010 S. Venerina (CT) (IT)
(72) Inventor: Rizza, Luisa, 95010 S. Venerina Catania (IT); Munafò, Salvatore, 95010 S. Venerina Catania (IT)
(74) Representative: Banfi, Paolo

(57) **Abstract**

The present invention refers to new oral pharmaceutical, nutraceutical, health foods and medical device compositions based on polysaccharides from Opuntia Ficus Indica cladodes in fixed or therapeutic combination with other actives and/or with other substances useful for the prevention or the of treatment of GERD (Gastro-Esophageal Reflux Disease), gastric esophagitis and related diseases (e.g. dyspepsia, esophagitis, esofageal tumour, gastrointestinal symptoms, chronic pharyngitis, Barrett's esophagus, esophageal adenocarcinoma, GERD-related pulmonary symptoms).

Said compositions, which have synergic action with substances useful for the treatment of the same diseases, allow to reduce the effective dose improving its therapeutic index.

## Description

### FIELD OF THE INVENTION

. The current invention refers to pharmaceutical, nutraceutical, health foods and medical device compositions for the treatment and the prevention of upper gastrointestinal tract diseases, as well as GERD (GastroEsophageal Reflux Disease), gastric esophagitis and related diseases.

. In particular, this invention provides an oral composition comprising of polysaccharides from Opuntia Ficus Indica cladodes in fixed or therapeutic combination with other actives and/or substances useful for the treatment and the prevention of GERD, gastric esophagitis and related diseases.

### BACKGROUND OF THE INVENTION

**.** Esophageal reflux acts in physiological conditions in everybody, in particular in newborns, but when it acts too frequently overcoming the esofageal mucosa defence mechanisms, it becomes a disease named GERD.

. GERD is the most common gastric disease and is caused by frequent gastric or enteric reflux in the esophagus which leads reflux esophagitis, a very common disease with typical esophageal mucosa inflammation and lesions (erosions and small ulcers) due to the gastric or enteric juice reflux in the esophagus.

. In GERD the reflux causes symptoms similar to esophagitis, but not to lesions.

. Normally between esophagus and stomach a pressure gradient inhibits the back flow of gastric contents in the esophagus.
The most important anatomical structure in maintaining the pressure gradient is the LES (Lower Esophageal Sphincter), a muscular ring at the esophagus and stomach union, which relaxes, in physiological conditions, during swallowing.

. In physiological conditions, when gastric (acid) substances reflux in the esophagus, the esophageal peristalsis (esophageal muscle contractions) sends them back to the stomach, and their acid residues are buffered, and so neutralized, by the saliva swallowed.

. GERD consists of a similarity between the pressure in the esophagus and the stomach. This could be due to one or more factors such as:
- lowered or absent LES tone;
- LES relaxing not swallowing related;
- reduced elimination of acidic material caused by abnormal esophageal peristalsis;
- abnormal function of cardias, the valve between stomach and esophagus that inhibits, in physiological conditions, the reflux of gastric contents in the esophagus.

. The imperfect cardias closure may be due to different causes, such as for instance obesity, smoking, multiple pregnancies, hard physical efforts, some pharmacological treatments or hiatus hernia.

. The serious types of reflux esophagitis may lead to esophagus stricture (stenosis) caused by esophagitis healing at the recovery, or to precancerous lesions ("Barrett's esophagus", esofageal adenocarcinoma) that produce an esophageal mucosa metaplasia, taking the typical appearance of gastric or duodenal mucosa. In some patients other factors are involved such as lowered saliva secretion, exaggerated acidic secretion, delayed gastric emptying, biliary salts or pancreatic enzymes reflux.

. These diseases are related to heartburn (hard retrosternal burning), frequent burps and food or acid regurgitation in the mouth. These symptoms can be connected to other symptoms affecting the respiratory system (chronic cough, asthma, hoarseness, chronic laryngitis), or affecting teeth (enamel loss) or the ear (otalgia).

. GERD and reflux esophagitis are currently treated, in the case of acidic reflux, with: 1) proton pump inhibitors (PPI, e.g. omeprazole, lansoprazole) and H₂ receptor antagonists (e.g. cimetidine and ranitidine), that reduce the acidic gastric secretion and consequently the reflux; 2) prokinetic agents (e.g. metoclopramide, domperidone, laevosulpiride), that increase the cardias valve tone and improve the gastric emptying; 3) antacid agents (e.g. bicarbonate, magnesium and aluminium hydroxide) and cytoprotective agents (e.g. sucralfate), that reduce gastric acidity and protect esophageal mucosa.

. Very few drugs are useful in case of non acid (enteric) regurgitation.

. Dietary, postural and behavioural practices can help reduce or prevent symptoms. Examples include avoiding alcohol, excessively large meals or very fatty, spicy or irritant foods (mint, chocolate, coffee, tea), or by sleeping with a pillow raised of some centimetres combined with pharmacological therapy.

. The pharmacological therapy doesn't work on the cause of the disease (the cardias malfunctioning) yet, and then it must be taken for very long periods or as long as one lives, because almost all patients complain of disease recurrence at the therapy suspension.

. The imperfect cardias valve closure may be corrected with surgery. This results in a complete recovery but if it is not well performed it may cause new symptoms in addition to the existing disease. Moreover, the surgical treatment is intended for young subjects, in excellent general condition, in need of continuous high doses therapy. Recently Dr. Higa got ready a new surgical method able to reduce GERD, but this new technique is not very widespread (http://www.medwatchtoday.com/1113.htm).

. There are not clear indications for choosing one or another pharmacological therapy for treating GERD or reflux esophagitis: physician chooses in general on the basis of his personal experience. Consequently, the choice of the therapy is very personal and not objective, in other words, it may be possible that a physician has to try more than one pharmacological therapy before finding the best fitted therapy to the patient's physiology.

. For patients with mild or occasional symptoms antacid agents, prokinetic agents and alginates are used, while in the most serious cases are used H₂ antagonists (anti H₂) and proton pump inhibitors (PPI).

. *Step* up clinical protocols - where less effective drugs are gradually combined with or replaced by more effective actives, on the basis of symptomatology - and *step down* clinical protocols - where the therapy begins with very effective actives such as proton pump inhibitors and continues with less effective actives, are provided for (Bytzer P., Goals of therapy and guidelines for treatment success in symptomatic gastroesophageal reflux disease patients, Am. J. Gastroenterol. 2003; 98(3) Suppl: S31-S39).

. PPIs are really effective actives, but their continuous use (which is needed in reflux esophagitis therapy) revealed serious side effects related to the long-term therapy with this class of actives e.g. risk of hip fracture due to calcium malabsorption through induction of hypochlorhidya (Yang e altri, Long term proton pomp inhibitor therapy and risk of hip fracture, JAMA 2006, 296(94): 2947-2953) and muscle diseases, such as polymyositis and rabdomiolisis (Clark et al., Myopathy including polymyositis: a likely class adverse effect of proton pump inhibitors?, Eur. J. Clin. Pharmacol., 2006 Jun, 62 (6) : 473-479).

. Various other PPI side effects were analysed in a *review* on the basis of drug control data pointed out in Spain from the 1^{th} January to the 31^{th} December 2004 by Salquiero et al. (Salquiero e altri, Safety profile of proton pump inhibitors according to the spontaneous reports of suspected adverse reactions, Int. J. Clin. Pharmacol. Ther., 2006 Nov, 44 (11): 548-556).

. Alginates, which have a physical mechanism of action (gelling and foaming at the acidic gastric pH, in presence of HCO₃) are safer than PPIs, but are not useful in the treatment of non acid esophageal reflux (Zentlin e altri, An evaluation of the antireflux properties of sodium alginate by means of combined multichannel intraluminal impedance and pH-metry, Aliment Pharmacol Ther. 2005; 21: 29-34).

**.** On the basis of what has been described before and on the increase in diseases such as GERD, reflux esophagitis and related diseases (dyspepsia, esophagitis, esophageal tumour, gastrointestinal disorders, chronic pharyngitis, Barrett's esophagus, esofageal adenocarcinoma, pulmonary dysfunctions GERD related) the finding of secure and effective treatment for the prevention and the therapy for these diseases is still relevant, at the same time, avoiding that the patient has to undergo different treatment to obtain an effective therapy.

### SUMMARY OF THE INVENTION

. New pharmaceutical, nutraceutical, health foods and medical device compositions able to co-operate in a synergistic way with actives useful in the prevention and therapy related with acid and not acid esophageal reflux diseases and its clinical complications are now identified. These compositions are characterised by containing a polysaccharides extract from Opuntia Ficus Indica cladodes.

. It is to be noted that for *medical device* in this description an "Active medical device" (class I) is intended in accordance with the Directive 93/42/EEC Annex IX classification.

. Opuntia Ficus Indica (Nopal) is a plant belonging to Cactaceae, native to Mexico and to United States south-west, but widespread even in self-sown Mediterranean vegetation.

. Fruits and younger cladodes (more tender) have been used since ancient times for dietary use (the cladodes are used fresh, brined, pickled, candied, as jam and even as forage) or to prepare alcoholic drinks.

. In popular medicine the plant is used in many ways: fruits are thought to be astringent and were used by sailors, because of their vitamin C wealth, to prevent scorbutus; young cladodes are used as cataplasm - oven wormed - as emollient agents; the use of cladodes pulp on coetaneous wounds and ulcers is an excellent anti inflammatory, reepithelialising and healing remedy: it is an old traditional Sicilian remedy still used nowadays in the country; the flowers decoction has diuretic properties.

. More recently it was observed that: 1) Opuntia Ficus Indica fruits have marked antioxidant properties (Tesoriere and al., Supplementation with cactus pear (Opuntia ficus-indica) fruit decreases oxidative stress in healthy humans: a comparative study with vitamin C, Am J Clin Nutr. 2004 Aug; 80(2): 391-395); 2) an Opuntia Ficus Indica extract is effective in alcohol hangover therapy (Wiese J and al., Effect of Opuntia Ficus Indica on symptoms of the alcohol hangover, Arch Intern Med. 2004 Jun 28; 164(12): 1334-1340); 3) the high polysaccharidic fraction concentration of Opuntia cladodes, essentially formed by a polymer of galactose, arabinose and other sugars, has swallowed fat and sugars ligand properties ( so making them impossible to be absorbed) leading to good results on glyco-lipidic metabolisms and on metabolic syndrome; 4) Opuntia Ficus Indica cladodes mucilages and pectins proved to be gastroprotective in experimental animal models (Vazquez-Ramirez R. and al., Reversing gastric mucosal alterations during ethanol-induced chronic gastritis in rats by oral administration of Opuntia Ficus Indica mucilage, World J. Gastroenterol. 2006 Jul 21; 12(27): 4318-4324).

. Trombetta and al. (Trombetta D. and al., Effects of polysaccharides from Opuntia Ficus Indica (L.) cladodes on the healing of dermal wounds in the rat, Phytomedicine, 13 (2006) 352-358) observed that polysaccharides extract from Opuntia Ficus Indica cladodes have cutaneous healing effect acting on cellular matrix and modulating laminin laying down.

. It was now unexpected observed that polysaccharides extracted from Opuntia Ficus Indica cladodes act in a synergistic way with substances useful in treating GERD, the complications associated with the disease and its prevention.

. The present invention related to pharmaceutical, nutraceutical, health foods and medical device formulations suitable for oral administration comprising polysaccharides extract from Opuntia Ficus Indica cladodes in fixed or therapeutic combination with other substances whose efficacy is increased from the claimed compositions (e.g. antacid agents, proton pump inhibitors, H₂ antagonists, prokinetic agents, sodium bicarbonate, alginic acid or alginates, chitosanes, aluminum hydroxide, calcium or magnesium carbonate, antioxidant and anti-inflammatory flavonoides extracted from plants as well as those extracted from olive leaves or Capparis Spinosa buds, anthocianosides from red oranges, black rice or from other natural sources and their combinations) useful for prevention and therapy of the same diseases such as GERD, reflux esophagitis and related diseases (dyspepsia, esophagitis, esophageal tumour, gastrointestinal disturbs, chronic pharyngitis, Barrett's esophagus, esophageal adenocarcinoma, pulmonary dysfunctions GERD related) and their use in preventing and treating these diseases.

. These compositions enable advantageously the reduction the dose of pharmacologically acting substances because of their efficacy is strengthened by the synergistic action demonstrated by Opuntia Ficus Indica polysaccharides. This determines a higher grade of therapeutic safety, a very important for children, pregnant women and elderly people or in preventing the diseases mentioned above to coincide, for example, with long therapy with NAIDS (Non Steroidal Anti Inflammatory Drugs).

. This allows for effective and safe topical targeted therapy, able to prevent or cure gastrointestinal tract diseases - as well as dyspepsia, esophagitis, esophageal tumour, Barrett's esophagus, esophageal adenocarcinoma, pulmonary dysfunctions GERD related - even in combination with other therapies for the same diseases.

. A first object of the invention is therefore that of providing pharmaceutical, nutraceutical, health foods and medical device oral compositions comprising polysaccharides from Opuntia Ficus Indica cladodes to be used in fixed or therapeutic combination with other substances useful in preventing or curing upper gastrointestinal tract diseases, as stated in the main attached claim.

. A second object is the use of said compositions for the production of a medicament to treat GERD, dyspepsia, esophagitis, esophageal tumour, gastrointestinal disturbs, chronic pharyngitis, Barrett's esophagus, esophageal adenocarcinoma, pulmonary dysphunctions GERD related, in fixed or combination therapy with other substances useful to prevent and to cure the same diseases, whose efficacy is increased from the synergistic action determined by the claimed compositions (e.g. antacid agents, proton pump inhibitors, H2 antagonists, prokinetic agents, sodium bicarbonate, alginic acid or alginates, chitosanes, aluminium or magnesium hydroxide, calcium or magnesium carbonate, antioxidant and anti-inflammatory flavonoides extracted from plants as well as those extracted from olive leaves or Capparis Spinosa buds, anthocianosides from red oranges, black rise or from other natural sources and their combinations).

### A BRIEF DESCRIPTION OF THE FIGURES

. Further characteristics and advantages of this invention will become more evident from the following description of several embodiments given as non-limiting examples with reference to the figures, wherein, with regard to the claimed compositions:
- Figure 1A represents a drawing of cladodes and fruits Opuntia Ficus Indica;
- Figure 2 represents a scheme of the *in vitro* model of mucoadhesion;
- Figure 3 represents in graph the comparison of the synergistic action of polysaccharides extracted from *Opuntia Ficus Indica* cladodes with sodium alginate in comparison to the same components applied one by one in an *in vitro model on epithelial buccal mucosa cells;*
- Figure 4 represents a table in which the percentage values of mucoadhesion (inhibition percentage of lectin binding after treatment) for each tested substances compared in Fig. 3.

### DETAILED DESCRIPTION OF THE INVENTION

. This invention provides oral pharmaceutical, nutraceutical, health foods and medical device compositions for treating and preventing esophageal reflux and GERD and related diseases (dyspepsia, esophagitis, esophageal tumour, gastrointestinal disturbs, chronic pharyngitis, Barrett's esophagus, esophageal adenocarcinoma, pulmonary dysfunctions GERD related) comprising polysaccharides extracted from Opuntia Ficus Indica cladodes, eventually supported by maltodextrins up to 50% , in fixed or combination therapy with actives or substances useful for the treatment or prevention of the same diseases.

. The compositions claimed may be liquid or solid, able to be administered *per* os (e.g. oral solutions, oral suspensions, oral emulsions, tablets, powders and tablets to prepare extemporarily oral solutions, suspensions or emulsions).

. These compositions may moreover include preservatives, ionic and non ionic buffered agents, and additives normally useful in preparing oral compositions.

. For liquid oral compositions, as well as solutions, suspensions or emulsions, the solvent is preferably water or an aqueous solution of one or more components consistent with the oral use.

. The actives or substances useful for treating or preventing upper gastrointestinal tract diseases in fixed or therapeutic combination with the Opuntia Ficus Indica cladodes extract of the claimed compositions can be chosen, as non-limiting examples, from among the group composed of: 1) other substances already used for the cure of the same diseases such as: proton pump inhibitors, H2 antagonists, prokinetic agents, antacid agents, sodium bicarbonate, alginic acid or alginates, chitosanes, sodium bicarbonate and their combinations; 2) other natural substances such as: antioxidant and anti inflammatory flavonoides from plants, as well as those from olive leaves, Capparis Spinosa buds, antocyanosides from red oranges, black rice or other natural sources and their combinations; 3) combinations of substances in 1) and 2).

. The methods to obtain the pharmaceutical or nutraceutical claimed compositions are known in the technical field. Moreover, Opuntia Ficus Indica extracted polysaccharides may be present from 0.5 to 50% of the total compositions, while the other actives may be present from 1 to 30%, the remaining being conventional preservatives.

. The single substances comprised in the compositions of the invention are already applied in the dietary field, so they are safe even for delicate patients like elderly people, children or pregnant women.

. This invention even includes the use of the described compositions for treating and preventing the upper gastrointestinal tract diseases administering to a subject therapeutically effective amount of the compositions as defined before, in fixed or therapeutic combination with other substances useful for treating or preventing the same diseases.

. The pathological conditions that may be effectively treated with the compositions of the present invention there are: gastroesophageal reflux, GERD, dyspepsia, esophagitis, and esophageal tumour, gastrointestinal disturbs, chronic pharyngitis, Barrett's esophagus, esophageal adenocarcinoma, pulmonary dysfunctions GERD related.

. Non limiting examples of compositions according to the invention are reported in the following tables.

### Example 1: Compositions

### 1.1 Oral suspension Composition 1

| **Components** | **% w/v** |
|---|---|
| Polysaccharides from Opuntia Ficus Indica cladodes | 2 g (1 ÷ 20g) |
| Water | up to 100 ml |

### 1.2: Oral suspension Composition 2

| **Components** | **% w/v** |
|---|---|
| Sodium Alginate | 5 g |
| Sodium Bicarbonate | 2.67 g |
| Polysaccharides from Opuntia Ficus Indica cladodes | 2.0 g (1 ÷ 20g) |
| Water | up to 100 ml |

### 1.3: Powder (bags) Composition 1

| **Components** | **% w/v** |
|---|---|
| Sodium Alginate | 10 - 20 g |
| Sodium Bicarbonate | 5 -10 g |
| Polysaccharides from Opuntia Ficus Indica cladodes | 1 - 20g |

### 1.4: Powder (bags) Composition 2

| **Components** | **% w/v** |
|---|---|
| Sodium Alginate | 10 - 20 g |
| Sodium Bicarbonate | 5 -10 g |
| Olive leaves extract | 5 - 20 |
| Polysaccharides from Opuntia Ficus Indica cladodes | 1 - 20g |

### 1.5: Capsule Composition 1

| **Components** | **mg/capsule** |
|---|---|
| Cimetidine | 200 mg |
| Opuntia Ficus Indica cladodes extract | 100-200 mg |
| Olive leaves extract | 100-200 mg |
| Lactose | up to capsule filling |

### 1.6: Capsule Composition 2

| **Components** | mg/capsula |
|---|---|
| Ranitidine chloridrate | 84 mg |
| Alginic acid | 500.0 mg |
| Opuntia Ficus Indica cladodes, extract | 200 mg |
| Olive leaves extract | 200 mg |
| Sodium bicarbonate | 165.25 mg |
| Magnesium stearate | 0.75 mg |
| Filler | 350 mg |

### Example 2: Test in vitro

### In vitro determination of mucoadhesion capability of polysaccharides from Opuntia Ficus Indica cladodes alone and in combination with alginic acid on buccal mucosae epithelial cells.

. In order to assess the mucoadhesive capability of polysaccharides from Opuntia Ficus Indica cladodes it was used the in vitro mucoadhesion assay described by Patel (Patel D. and al., An in vitro mucosal model predictive of bioadhesive agents in the oral cavity. J Control Release, 1999, 61(1-2) : 175-183) on suspended epithelial cells from buccal mucosa. In this test the mucoadhesion (adhesive capability of the tested substances in terms of binding to the mucosal cells) is determined as a function of binding lectin-membrane glycoprotein inhibition determined by the cellular surface hiding due to the mucoadhesive substance.

### Materials and Methods

. A treatment of mucosal cells with biotinylated lectin and streptavidin peroxidase, determines a yellow colour of the sample; a pre-treatment of mucosal cells with substance whose mucoadhesive is to be detected, (incubation at 30°C, for 15 minutes before the treatment with lectin), whenever said substance is mucoadhesive, the lectin binding site be hidden, reducing the final yellow colouring intensity.

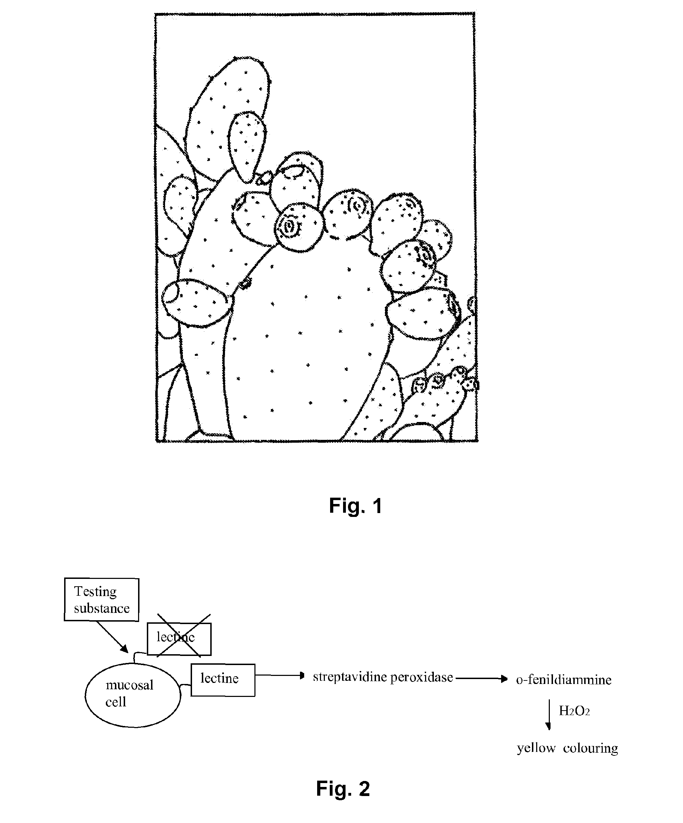

. Lectin is a protein contained in some plants of Leguminosae (*Canavalia ensiformis),* with high tendency to bind glucosidic and mannosidic residues of membrane glycoproteins; biotin (vitamin H) is artificially bonded to lectin because it binds to streptavidin peroxidase that is added to cellular suspension to obtain the complex protein-glucose-lectin-biotin-streptavidin peroxidase. Adding o-phenylendiamine dihydrochloride (o-pd) and hydrogen peroxide to the cellular suspension, if in the environment streptavidin peroxidise is present (and thus the complex protein-glucose-lectin-biotin-streptavidin peroxidise) o-pd is oxidized to 2,3-diaminophenazine, and the sample becomes then yellow (Fig. 2).
The reaction is then blocked after 1 min adding H₂SO₄ 1M. The colour intensity is read at the spectrophotometer and it will be proportionally related to binding between lectin and glycosidic residues on cellular membrane.

. Buccal mucosa cells were obtained from healthy volunteers , male and female, by gently scraping the inner cheeks of the oral cavity with a wooden spatula. The cells were then pulled and suspended in 10 ml isotonic Tris 0.05M (TBS) pH 7.6 (Patel D. and al., An in vitro mucosal model predictive of bio-adhesive agents in the oral cavity. J Control Release, 1999, 61(1-2) : 175-183). A suspension sample was then added of O.lml trypan blue 0.5% (w/v) and the total number of cells was determined using an haemocytometer: each sample contained 48 × 10⁴ buccal mucosa cells. Polysaccharides from Opuntia Ficus Indica cladodes and sodium alginate were solubilised NaCl 0.9% isotonic solution at 0.5% w\v and these solutions were then used for the pre-treatment of the test on buccal mucosa cells. Results were expressed in percentage of tested substance and mucosa cells binding, inversely proportional to percentage of reduction of sample colouring in comparison to the control. The whole experiment was repeated 5 times.

. Statistical analysis of results was performed by means of one-way ANOVA followed by Bonferroni post hoc test.

### Results

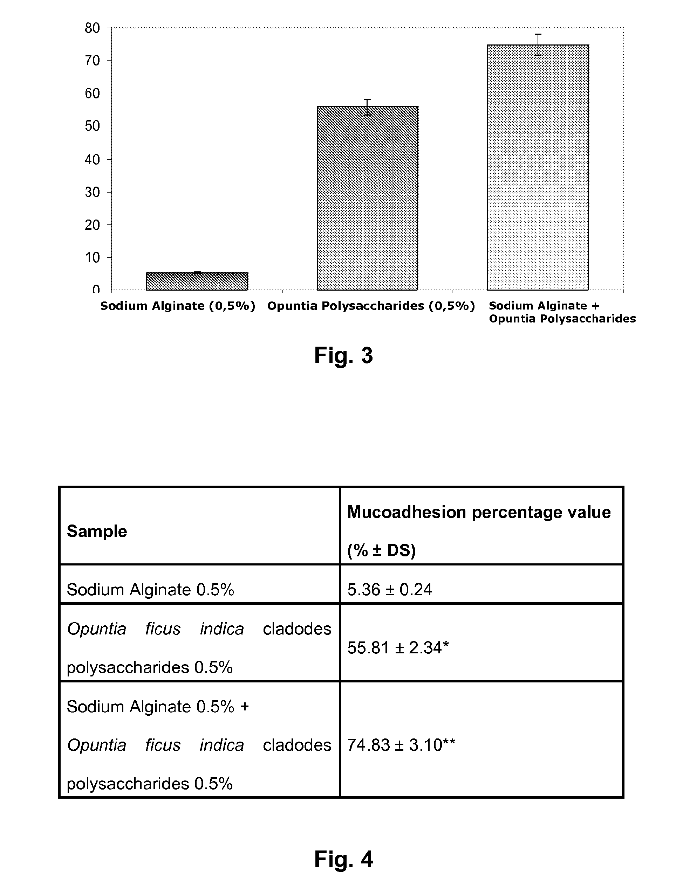

. As depicted in Figs. 3 and 4, the results obtained with the *in vitro* mucoadhesion assay show an Opuntia Ficus Indica polysaccharides mucoadhesion on buccal mucosa suspended cells percentage of 55.81%, significantly higher than that observed with sodium alginate (mucoadhesion 5.36%; p<0.05). Furthermore it was observed that by adding sodium alginate to Opuntia ficus Indica cladodes polysaccharides the mucoadhesive properties reach a percentage value of 74.83%.

### Conclusions

. The results obtained with the mucoadhesion *in vitro* assay show that Opuntia Ficus Indica cladodes polysaccharides have a mucoadhesive character much higher than observed with sodium alginate. It was unexpected observed that the combination of these substances determines a synergistic effect that guarantees a strengthened mucoadhesive activity.

### Example 4: In vivo evaluation of antiulcer activity of sodium alginate /antacid/Opuntia Ficus Indica cladodes polysaccharides/Olea Europeae leaves extract composition.

. This study was performer to assess the antiulcer activity of the combination of sodium alginate/antacid, Opuntia Ficus Indica cladodes polysaccharides and Olea Europeae leaves extract in an animal model of ethanol and Indomethacin induced ulcer in rats.

### Materials and methods

. The experimental protocol allowed for the evaluation of the efficacy of 5 compositions: alginate/antacid combination (A), Opuntia Ficus Indica cladodes polysaccharides (B), Olea Europeae leaves extract (C), alginate\antacid\Opuntia Ficus Indica cladodes polysaccharides combination (D) and alginate\antacid\Opuntia Ficus Indica cladodes polysaccharides\ Olea Europeae leaves extract combination (E).

. Each sample was obtained by suspending in water second the percentage concentration reported in Table 1 and administered with an oral probe (1 ml 100g weight).

**Table 1: Compositions compared in the animal in vivo model**

| | Compositions (% w/v) | | | | |
|---|---|---|---|---|---|
| Components | A | B | C | D | E |
| Sodium Alginate | 5 | - | - | 5 | 5 |
| Sodium Bicarbonate | 2.67 | - | - | 2.67 | 2.67 |
| Opuntia Ficus Indica cladodes polysaccharides | - | 2 | - | 2 | 2 |
| Olea Europeae leaves extract | - | - | 1 | - | 1 |

. The experiment was performed on Sprague-Dawley (Rattus norvegicus) adult rats, male and female, weight ranging of 180 and 200 g. The animals were exposed to natural light in controlled temperature (22 ± 2°C) and humidity (60 % ± 4) conditions, and fed with a standard (Morini Mill rat GLP) and water *ad libitum* for one week. The animals were then deprived of food 24 hours and of water 2 hours before the start of the experiment (F. Vitali, Antiulcer potential of a standardized extract of red orange juice in the rat, Int J Food Properties, 10, 331-344, 2007).

. ***Experimental model of inducing of ulcer with ethanol***

. The animals were randomly divided into 6 groups (1-6) with 6 animals in each. Group 1 was used as control and was treated with the vehicle (distilled water 10 ml/Kg weight) and, after 1 hour, with the ulgerogenic agent: absolute ethanol Iml/rat; group 2 received per os sample A (767 mg /kg weight), group 3 received per os sample B (200 mg /kg weight); group 4 received per os sample C (100 mg /kg weight); group 5 received per os sample D (967 mg /kg weight) and group 6 received per os sample E (1067 mg /kg weight). After 1 hour the animals of groups 2-6 received, from samples A-E, 1 ml/animal of absolute ethanol, to induce the ulcer. 1 hour after the treatment with ethanol, all animals were anesthetised with pentobarbital and killed. The stomach of each animal was then removed, opened along the greater curvature and gently rinsed with NaCl 0.9% solution, not removing the superficial mucosa.

### Experimental model of inducing of ulcer with Indomethacin

. The animals were randomly divided into 6 groups (1-6) with 6 animals in each. Group 1 was used as control and was treated with the vehicle (distilled water 10 ml/Kg weight) and, after 1 hour, with the ulgerogenic agent: Indomethacin (suspension in distilled water containing carboxymethylcellulose 1% at the dose of 0.05 g/Kg, 1 ml/Kg weight s.c.).

. Groups 2-6 were treated, as described for the model of ulcer induced by absolute ethanol, with samples A-E, and after 1 hour, with Indomethacin (suspension in distilled water containing carboxymethylcellulose 1% at the dose of 0.05 g/Kg, 1 ml/Kg weight s.c.). 5 hours after the treatment with Indomethacin, the animals were anesthetised with pentobarbital and killed. The stomach of each animal was then removed, treated and analysed as described in the experiment with ethanol.

### Evaluation of the efficacy of the tested compositions.

. Gastric mucosal surface was analysed with macroscopic observation. In particular, the length (mm) of each lesion on the mucosa was determined using an arbitral evaluation (Table 2: 5 scores for ethanol induced ulcer; Table 3: 6 scores for Indomethacin induced ulcer).

. For both the experimental models it was then calculated, for each stomach, the ulceration index (U.I), adding the points assigned to the ulcer observed, and the Ulceration Inhibition Percentage (P.I.U.): [(U.I._{control} - U.I.ₛₐₘₚₗₑ) **\**U.I._{Control}] *100. The significance of the data, as arithmetic media ± SEM for each group, was then analysed by means of Kruskal-Wallis followed by Dunn's test. A p<0.05 value was considered significant different.

. The evaluation scheme of the mucosa state for the 2 models is reported in Tables 2 and 3.

### Results

. In both the experimental models the Ulceration Inhibition Percentage (P.I.U.) obtained in group D (treated with the sodium alginate/antacid/Opuntia Ficus Indica cladodes polysaccharides combination) was higher than that one obtained with the treatment with the singular components of the same combination and also higher than the summary of their P.I.U.s, revealing a synergistic effect determined by the combination of the substances used. Unexpectedly, moreover, by adding the Olea Europeae leaves extract (group E) to the said combination the P.I.U. increases more than the summary of Olea Europeae leaves extract singular component and sodium alginate/antacid/Opuntia Ficus Indica cladodes polysaccharides combination P.I.U.s, revealing a new synergistic effect between them.

**Tab. 2 Score Evaluation System (1-5 scores) of the ulcer seriousness in the ethanol model**

| Score | Lesion length (mm) |
|---|---|
| 1 | 1-50 mm |
| 2 | 51-100 mm |
| 3 | 101-150 mm |
| 3 | 151-200 mm |
| 5 | > 201 mm |

**Tab. 3 Score Evaluation System (1-5 scores) of the ulcer seriousness evaluation in the Indomethacin model**

| Score | Description |
|---|---|
| 0 | No ulcer |
| 0.5 | punctiform ulcer (diameter ≤ 2 mm) or thready ulcer (length ≤ 5 mm) |
| 2 | punctiform ulcer (diameter > 2 mm) ≤ 5 mm) or thready ulcer (length > 5 mm ≤ 10 mm) |
| 4 | punctiform ulcer (diameter > 5 mm ≤ 10 mm) or thready ulcer (length > 10 mm) |
| 6 | punctiform ulcer (diameter > 10 mm) |

### Conclusions

. The in vivo test revealed an unexpected synergistic potentiation determined by Opuntia Ficus Indica cladodes polysaccharides on the sodium alginate/antacid effect. This synergistic effect is moreover newly potentiated adding the Olea Europeae leaves extract. Practically, the ulcer lesions observed in animals treated with conventional treatments (sodium alginate plus sodium bicarbonate) revealed a very high score, while in the animals treated with the combination described in this invention had very low ulcer score. More specifically, very low ulcer scores demonstrate that the claimed combination protects gastric mucosa with an unexpected synergistic effect between its singular components.

. From these results it can be deduced that it is possible using these substances in fixed or therapeutic combination to reduce the dose of the actives useful in preventing or treating upper gastrointestinal tract diseases, increasing in a synergistic their efficacy and reducing their unwanted side effects.

### Example 5: In vivo evaluation of the efficacy of the sodium alginate/antacid/polysaccharides cladodes Opuntia Ficus Indica/Olea Europeae leaves extract combination in reducing the gastroesophageal reflux surgically induced.

**. This study was performed to evaluate the efficacy of the sodium alginate/antacid/polysaccharides cladodes Opuntia Ficus Indica combination added with Olea Europeae leaves extract in reducing the gastroesophageal reflux surgically induced in rats.**

. The reducing of gastroesophageal reflux was quantified on the base of the esophageal ulcers observed in animals treated with the combinations in study after the surgically inducing the gastroesophageal reflux.

. Adult Sprague-Dawley rats (Rattus norvegicus), male and female, weighting in the range of 180 - 200 grams, were randomized 6 groups (1-6), each of 6 animals. Referring to the experimental protocol described by Min (Min et al.: The effects of apigenin-7-O-β-D-glucurono-pyranoside on reflux oesophagitis and gastritis in rats, Autonomic & autacoid Pharmacology 2005 Ju1;25(3):85-91), the animals were anesthetised and both the pylorus and limiting ridge (transitional region between the fore stomach and corpus) were then simultaneously ligated. Group 1 (not treated) was the positive control. Groups 2-6 were intraduodenally treated with compositions A-E immediately after the ligation of pylorus and the limiting ridge, using the same dosages reported in the chemically induced ulcer models already described. Afterwards, a longitudinal cardiomyotomy of about 1 cm length across the gastro-oesophageal junction was performed to enhance reflux from the stomach contents into the esophageal body. After 6 h, the animals were killed and the esophagus was harvested and then the lesions total area was evaluated using the scoring reported in Tab. 4.

. The final ulceration index (U.I.) and the P.I.U. for each group were calculated as described before.

**Tab. 4 Score Evaluation System (5 scores) of esophageal mucosa lesions gravity**

| **Score** | **Total area (mm²)** |
|---|---|
| 0 | No visibile lesions |
| 1 | A few lesions |
| 2 | Total area of the lesions ≤30 mm² |
| 3 | Total area of the lesions ≥ 30 mm² |
| 4 | Perforation |

### Results

. As observed in the *in vivo* models described before, even in this model the Ulceration Inhibition Percentage (P.I.U.) obtained with group D (treated with the sodium alginate/antacid/Opuntia Ficus Indica cladodes polysaccharides) was higher than that observed in the experimental groups treated with the singular components of the same combination and even higher than the summary of their P.I.U.s, revealing a synergistic effect determined by the sodium alginate/antacid/Opuntia Ficus Indica cladodes polysaccharides combination even in reducing the gastroesophageal reflux. The further adding of Olea Europeae leaves extract to this combination (group E) increases the P.I.U. at a level higher than the simple summary of Olea Europeae leaves extract singular component and combination D P.I.U.s., confirming the synergistic effect between these substances that was already observed in the chemically induces gastric ulcer models.

### Conclusions

. Even this *in vivo* test revealed a synergistic potentiating determined by Opuntia Ficus Indica cladodes extract and sodium alginate/antacid combination, in reducing the gastroesophageal reflux. This effect is moreover increased in a synergistic way by the adding of Olea Europeae leaves extract.

. The observed data confirms that the combination of these substances increases in a synergistic way the effect of the actives useful in preventing and therapy of upper gastrointestinal tract, enabling reducing their effective dose and then the unwanted effects.

## Claims

1. Compositions comprising Opuntia Ficus Indica cladodes extract in combination with substances useful in the therapy of the upper gastrointestinal tract.

2. Compositions according to the claim 1, where the substances useful in the therapy of the upper gastrointestinal tract are chosen between Proton Pump Inhibitors (PPIs), H₂ receptors antagonists, prokinetic agents, antacid agents, cytoprotecting agents, anti-inflammatory agents and their combinations.

3. Compositions according to the claim 2, where said substances are chosen between: omeprazole, lansoprazole, cimetidine, ranitidine, metoclopramide, domperidone, laevosulpiride, magnesium and aluminium hydroxide, sodium bicarbonate, alginic acid or alginate salts, chitosanes, sucralfate, anti-inflammatory flavonoides extracted from extracted from Olea Europeae or from Capparis Spinosa, anthocianosides from red oranges, black rice or from other natural sources and their combinations.

4. Compositions according to any one of the claims 1 - 3 wherein the Opuntia Ficus Indica cladodes polysaccharides are comprised in quantities from 0.5% to 50%, whereas the other actives are comprised in quantities from 1% to 30% on the total amount of said compositions.

5. Compositions according to any one of the claims 1 - 4 useful for oral administration.

6. Compositions according to any one of the claims 1 - 5 comprising diluting agents, buffer and excipients useful for pharmaceutical, nutraceutical, dietary and healthy use.

7. Compositions according to any one of the claims 1 - 6 in form of: oral solution, oral suspension, oral emulsion, tablets, capsules, powders and bags for the extemporary preparation of oral suspensions or oral emulsions.

8. Compositions according to any one of the claims 1 - 7 for medical use.

9. Pharmaceutical, nutraceutical, dietary compositions consisting of:
| **Components** | **%** |
|---|---|
| Sodium Alginate | 5 g |
| Sodium Bicarbonate | 2.67 g |
| Opuntia Ficus Indica cladodes polysaccharides | 2.0 g (1 ÷ 20g) 2.0 g (1 ÷ 20g) |
| Water | Up to 100 ml |

10. Pharmaceutical, nutraceutical, dietary compositions consisting of:
| **Components** | **%** |
|---|---|
| Sodium Alginate | 10 - 20 g |
| Sodium Bicarbonate | 5 - 10 g |
| Opuntia Ficus Indica cladodes Polysaccharides | 1 - 20 g |

11. Pharmaceutical, nutraceutical, dietary compositions consisting of:
| **Components** | **%** |
|---|---|
| Sodium Alginate | 10 - 20 g |
| Sodium Bicarbonate | 5 - 10 g |
| Olea Europeae leaves extract | 5 - 20 g |
| Opuntia Ficus Indica cladodes, polysaccharides | 1 - 20 g |

12. Pharmaceutical, nutraceutical, dietary compositions consisting of:
| **Components** | mg/capsule |
|---|---|
| Cimetidine Opuntia Ficus Indica cladodes Polysaccharides | 200 mg 100 - 200 mg |
| Olea Europeae leaves extract | 100 - 200 mg |
| Lactose | up to capsule filling |

13. Pharmaceutical, nutraceutical, dietary compositions consisting of:
| **Components** | mg/capsule |
|---|---|
| Ranitidine hydrochloride | 84 mg |
| Alginic Acid | 500.0 mg |
| Opuntia Ficus Indica cladodes polysaccharides | 200 mg |
| Olea Europeae leaves extract | 200 mg |
| Sodium Bicarbonate | 165.25 mg |
| Magnesium Stearate | 0.75 mg |
| Filler | 350 mg |

14. Compositions according to any one of the claims 1 - 8 for use in the treatment or in prevention of upper gastrointestinal tract diseases.

15. Compositions according to claim 14, wherein said diseases are: gastroesophageal reflux, GERD, dyspepsia, gastrointestinal disturbs, pharyngitis, Barrett's esophagus, esophageal adenocarcinoma, GERD-related pulmonary symptoms.
